# EUROPÄISCHE PATENTSCHRIFT

(11) **EP 0 726 275 B1**
(45) Veröffentlichungstag und Bekanntmachung des Hinweises auf die Patenterteilung: **27.01.1999**
(21) Anmeldenummer: 96101315.8
(22) Anmeldetag: 31.01.1996
(51) Int. Cl.: C07K 14/00, C07K 16/44, G01N 33/94, G01N 33/531, C07D 403/12, C07D 403/06

(54) **Benzodiazepinproteinkonjugate und ihre Verwendung zur immunologischen Bestimmung von Benzodiazepinen**
Benzodiazepine protein conjugates and their use in benzodiazepine immunoassays
Conjugués protéiques de benzodiazépines et leur utilisation pour le dosage immunologique de benzodiazépines

(30) Priorität: 02.02.1995 DE 19503320
(43) Veröffentlichungstag der Anmeldung: 14.08.1996
(73) Patentinhaber: BOEHRINGER MANNHEIM GMBH, 68298 Mannheim (DE)
(72) Erfinder: Huber, Erasmus, Dr., D-86923 Finning (DE); Klein, Christian, Dr., D-82362 Weilheim (DE); Josel, Hans-Peter, Dr., D-82362 Weilheim (DE); Zink, Bruno, D-82449 Uffing (DE)

(56) Entgegenhaltungen:
- WO-A-90/15798
- WO-A-93/20067
- BIOCONJUGATE CHEMISTRY, Bd. 3, Nr. 1, 1992, WASHINGTON DC, US, Seiten 2-13, XP000261480 M. BRINKLEY: "A brief survey of methods for preparing protein conjugates with dyes, haptens, and cross-linking reagents"
- JOURNAL OF MEDICINAL CHEMISTRY, Bd. 37, Nr. 16, 5.August 1994, WASHINGTON DC, US, Seiten 2609-18, XP002002284 Y. ARANO ET AL.: "Maleimidoethyl 3-(tri-n-butylstannyl)hippurate: a useful radioiodination reagent for protein radiopharmaceuticals to enhance target selective radioactivity localization"

## Beschreibung

Die Erfindung betrifft Benzodiazepinproteinkonjugate, deren Herstellung, deren Verwendung als Immunogen, ein Verfahren zur Erzeugung von Antikörpern gegen Benzodiazepine und ein Immunoassay zum Nachweis von Benzodiazepinen unter Verwendung dieser Antikörper. Weiter sind Gegenstand der Erfindung neue Benzodiazepin-Linkerverbindungen, deren Herstellung und deren Verwendung zur Herstellung der neuen Konjugate.

Der analytische Nachweis von Verbindungen aus der Klasse der Benzodiazepine hat in den letzten 1 1/2 Jahrzehnten zunehmende Bedeutung erlangt. Benzodiazepine spielen eine große Rolle als Arznei und Beruhigungsmittel, können andererseits als Sucht- und Ausweichdrogen mißbraucht werden, spielen eine Rolle bei Vergiftungen, vor allem bei Mischintoxikationen und Suiciden. Ihr Nachweis ist auch von großer verkehrsmedizinischer Relevanz.

Deshalb besteht zunehmend Interesse an immunologischen Nachweismethoden zum schnellen, qualitativen und quantitativen Nachweis von Benzodiazepinen in Körperflüssigkeiten. Für Immunoassays notwendig sind kupplungsfähige Benzodiazepinlinker-Verbindungen ("Hapten-Linker-Verbindungen") zur Synthese von Benzodiazepinproteinkonjugaten ("Haptenproteinkonjugate"). Die Benzodiazepinproteinkonjugate werden einerseits als Immunogene zur Erzeugung von gegen Benzodiazepine gerichteten Antikörpern eingesetzt, Benzodiazepin-Enzymkonjugate können andererseits auch als enzymmarkierte Antigene oder als Polyhaptene in Immunoassays verwendet werden.

Benzodiazepinlinkerverbindungen und Benzodiazepinproteinkonjugate sind bekannt: z.B. EP-A-0 264 797, J. Pharm. Sci. 66, 235 (1977), Biochem. Pharm. Exp. Therapeutics 186, 167 (1973), J. Immunoassay 4, 135 (1983), US-P-4,243,654, US-P-4,046,636, US-P-4,777,169, US-P-4,043,989, WO-A-93/20067 und US-P-4,083,948. Die damit erzeugten Antikörper werden in Serumtests eingesetzt.

Ebenfalls bekannt ist die Verwendung von Maleimidgruppen in Linkern zur Anbindung von Haptenen an Proteine, bspw. aus WO-A 90/15798, J. Med. Chem. 37, 2609 (1994) und Bioconjugate Chem. 3, 2 (1992).

Eine Besonderheit bei Immunoassays zum Nachweis von Benzodiazepinen liegt darin, daß Antikörper benötigt werden, die nicht nur eine bestimmte Benzodiazepinverbindung, sondern die eine möglichst große Anzahl verschiedener relevanter Benzodiazepine erkennen können (hohe Kreuzreaktivität). Eine weitere Schwierigkeit zum Nachweis von Benzodiazepinen besteht dann, wenn diese in Urin nachgewiesen werden sollen. Hier treten Abbauprodukte (Metabolite) der Benzodiazepine auf (Clarke's Isolation and Identification of Drugs, 2nd ed., The Pharmaceutical Press, London 1986; R.C. Baselt, Disposition ofToxic Drugs and Chemicals in Man, 3rd ed., Year Book Medical Publishers Inc., 1989), insbesondere Amine, Glucuronide und Benzophenone, die durch die in einem Immunoassay verwendeten Antikörper ebenfalls erfaßt werden sollten. Die Aufgabe der Erfindung war es daher, Immunogene in Form von Benzodiazepinproteinkonjugaten zur Verfügung zu stellen, die als Immunantwort Antikörper mit einer möglichst hohen Kreuzreaktivität gegenüber relevanten Benzodiazepinen erzeugen, insbesondere auch gegen Benzodiazepinabbauprodukte im Urin.Ferner sollten Immunogene in Form von Benzodiazepin-Proteinkonjugaten zur Verfügung gestellt werden, die Antikörper erzeugen, deren Bindungsfähigkeit nur zu einem geringen Teil auf die Linkerteilstruktur des Konjugates gerichtet ist.

Die Aufgabe wird gelöst durch die Erfindung wie sie in den Ansprüchen beschrieben ist.

Gegenstand der Erfindung sind neue Benzodiazepinproteinkonjugate der Formel I in der R1 Wasserstoff, eine Methylgruppe oder R darstellt, R2 Wasserstoff, Hydroxy, oder eine OR-Gruppe darstellt, wobei entweder R1 oder R2 eine R-Gruppe enthält, R3 Halogen, NO2 oder NH2 darstellt, X Wasserstoff oder Halogen darstellt, wobei R eine Gruppe der Formel II darstellt, in der Z eine makromolekulare immunogen wirksame Trägersubstanz bedeutet und n=2 oder 3 ist.

### Bevorzugt ist n=2.

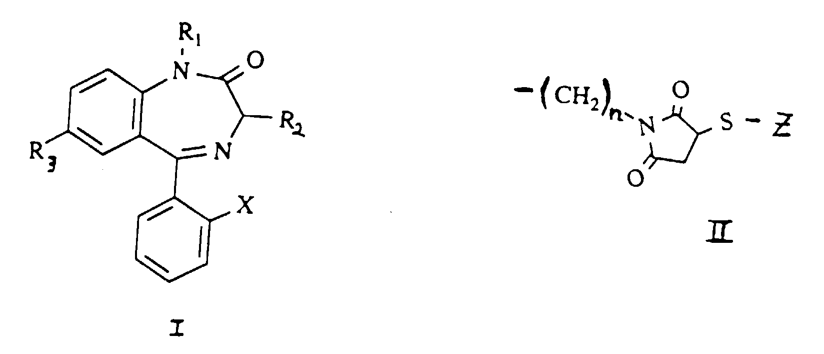

Makromolekulare Trägersubstanzen sind bevorzugt Polypeptide, beispielsweise KLH (key limpet haemocyanin), Edestin oder Rinderserumalbumin. Auch Enzyme, wie β-Galaktosidase, sind geeignet.

Unter Halogen wird Fluor, Chlor, Brom oder Jod verstanden. Bevorzugter Rest für R3 und X ist Chlor.

Bevorzugter Rest für R3 ist Halogen, insbesondere Chlor.

Ganz bevorzugte Verbindungen der Formel sind Verbindungen der Formel Ia und Ib. 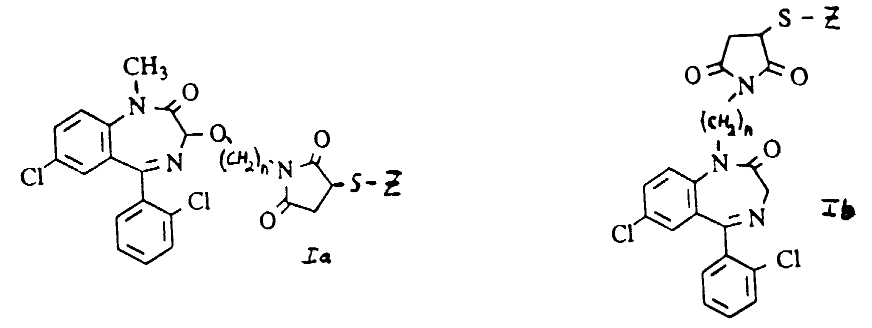 Die erfindungsgemäßen Konjugate werden als Immunogene zur Herstellung von Antikörpern gegen Benzodiazepine eingesetzt. Gegenstand der Erfindung ist ein Verfahren zur Erzeugung von Antikörpern gegen Benzodiazepine durch Immunisierung eines Säugetieres und Gewinnung der erzeugten Antikörper nach bekannten Verfahren, z.B. aus dem Serum oder der Milz, dadurch gekennzeichnet, daß ein erfindungsgemäßes Konjugat als Immunogen verwendet wird. Die Herstellung des Antiserums erfolgt nach bekannten Verfahren, vorzugsweise in Mäusen oder Schafen. Eine derartige Immunisierung ist beispielsweise in B. Dunkar, J. Agr. Food Chem. 38 (1990), 433 - 437 und N.H. Ogodrow, J. Agric Food Chem. 38 (1990), 940 - 946 beschrieben.

Ein weiterer Gegenstand der Erfindung sind Antikörper, die aufgrund Immunisierung mit den erfindungsgemäßen Benzodiazepinkonjugaten erhältlich sind. Diese Antikörper sind monoklonal oder bevorzugt polyklonal.

Die Herstellung von Antikörpern erfolgt durch die Immunisierung eines Versuchstieres mit dem Immunogen. Dieser Schritt des erfindungsgemäßen Verfahrens kann auf eine übliche, dem Fachmann bekannte Weise geschehen. Verzugsweise verabreicht man das Immunogen dem Versuchstier in Kombination mit einem Adjuvans. Besonders bevorzugt verwendet man als Adjuvans Freundsches Adjuvans oder Aluminiumhydroxid zusammen mit Bortadella pertussis. Die Immunisierung erfolgt vorzugsweise über mehrere Monate mit mindestens vier Immunisierungen in vier- bis sechswöchigem Abstand. Die Injektion des Immunogens erfolgt vorzugsweise intraperitoneal.

Aus so immunisierten Tieren werden B-Lymphozyten gewonnen, die mit einer permanenten Myelomzellinie fusioniert werden. Die Fusionierung erfolgt nach dem bekannten Verfahren von Köhler und Milstein (Nature 256, 1975, 495-497). Die hierbei gebildeten Primärkulturen von Hybridzellen werden in üblicher Weise, z.B. unter Verwendung eines handelsüblichen Zellsorters oder durch "limited dilution" kloniert. Es werden jeweils die Kulturen weiterverarbeitet, die in einem geeigneten Testverfahren, beispielsweise einem Enzym-Immuno-Assay (ELISA-Verfahren), positiv gegen das Benzodiazepin reagieren.

Die Antikörper zeigen eine hohe Kreuzreaktivität insbesondere gegenüber Temazepam, Oxazepam, Lorazepam, Bromazepam, Alprazolam und gegen Abbauprodukte wie Temazepam-Glucuronid oder -Benzophenon, Oxazepam-Glucuronid und Aminoflunitrazepam. Dabei weisen sie andererseits wenig Kreuzreaktivitäten mit der Linkerbrücke auf.

Ein weiterer Gegenstand der Erfindung ist die Verwendung des so erhaltenen Antikörpers in Immunoassays. Insbesondere werden die Antikörper an eine Markierung (z.B. ein Enzym, eine radioaktive Markierung oder ein Partikel wie Latex oder Metallsol) oder an einen weiteren Bindepartner (z.B. Streptavidin) oder an eine Festphase gebunden, verwendet.

Das Verfahren zur Bestimmung der Anwesenheit von Benzodiazepin oder Benzodiazepinmetaboliten unter Verwendung der erfindungsgemäßen Antikörper wird so durchgeführt, daß eine Probe, bevorzugt Urin, die auf Benzodiazepine untersucht werden soll, mit mindestens einem erfindungsgemäßen Antikörper, der gegebenenfalls markiert ist oder an eine Festphase gebunden ist oder mit einem weiteren Bindepartner an eine Festphase gebunden werden kann, kontaktiert wird und die Bildung des Antikörper-Benzodiazepin-Komplexes in einer geeigneten Weise, zum Beispiel über eine Markierung, bestimmt wird.

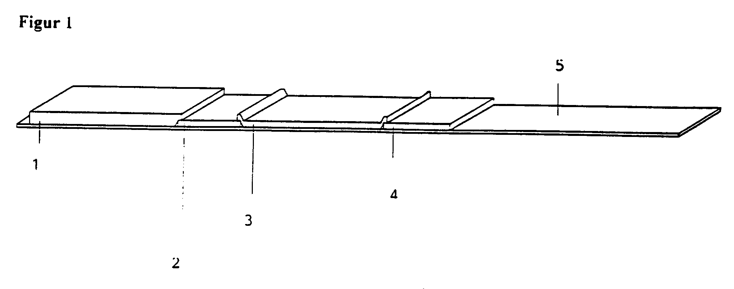

Bevorzugt wird der Immunoassay in Form eines heterogenen Immunoassays durchgeführt, besonders bevorzugt auf einem Chromatographieteststreifen, wie er beispielsweise in DE-OS 44 39 429 oder DE-OS 40 24 919 beschrieben ist (siehe Figur 1).

Ein solcher Teststreifen enthält bevorzugt auf einer Trägerfolie hintereinander angeordnete saugfähige Zonen: eine Analytaufgabezone (1), eine Konjugatzone (2), auf der ein markierter Bindungspartner und gegebenenfalls ein Bindungspartner mit einer spezifischen Bindestelle, z.B. Biotin, für die Abfangzone (3) untergebracht ist, eine Abfangzone (3), auf der ein Abfangreagenz für den Analyten oder ein Analytantikörper oder ein spezifisches Abfangreagenz für eine spezifische Bindestelle eines Bindungspartners, z.B. Streptavidin, aufgebracht ist, und eine Zielzone (4), in der nichtabgefangene Markierung gemessen wird.

Wird der Immunoassay nach einem Sandwich-Prinzip durchgeführt, bildet sich nach Aufgabe des Analyten auf die Aufgabezone in der Konjugatzone ein Komplex von Analyt mit einem markierten Antikörper, der über einen weiteren Antikörper, der entweder in der Festphasenzone festphasengebunden ist oder dort über ein spezifisches Bindungspaar, zum Beispiel Biotin-Streptavidin gebunden werden kann, dort immobilisiert wird.

Bei einem kompetitiven Test konkurrieren Analyt und markiertes Analytanalogon um einen Antikörper, der in der Abfangzone festphasengebunden oder dort über eine spezifische Bindungsstelle festphasengebunden werden kann.

Bevorzugt wird der Immunoassay nach einem IEMA analogen Testprinzip durchgeführt. Hier befindet sich in der Konjugatzone ein markierter Antikörper im Überschuß zum Analyten. Überschüssige markierte Antikörper werden in der Abfangzone durch Analytanaloga abgefangen, während markierte Analyt-Antikörper-Komplexe in der Zielzone nachgewiesen werden.

Als Markierungen kommen übliche Markierungen, wie Enzymmarkierung, Fluoreszenz-, Farbstoffmarkierungen in Frage, besonders Direktmarkierung, insbesondere Metallmarkierung, ganz besonders bevorzugt Goldmarkierung.

Der Immunoassay liefert schnelle und exakte Ergebnisse, die auch Abbauprodukte von Benzodiazepinen einschließt.

Ein weiterer Gegenstand der Erfindung ist ein Verfahren zur Herstellung der erfindungsgemäßen Benzodiazepinproteinkonjugate. Es ist dadurch gekennzeichnet, daß Benzodiazepin-Linkerverbindungen der Formel III mit einer Trägersubstanzverbindung, bevorzugt mit Polypeptidverbindungen, die mindestens eine Thiolgruppe enthalten, zu Verbindungen der Formel I umgesetzt werden. Dabei können die Polypeptidverbindungen von sich aus SH-Gruppen enthalten. Es können aber auch künstlich SH-Gruppen durch dem Fachmann bekannte Methoden in die Polypeptid-Verbindung eingeführt werden. Die Reste R3 und X und n der Formel III haben dabei die für Formel I gegebene Bedeutung, R1' ist Wasserstoff Methyl oder R', R2' ist Wasserstoff, Hydroxy oder eine OR'-Gruppe, wobei entweder R1' oder R2' eine R' Gruppe enthält und R' eine Gruppe der Formel IV bedeutet. 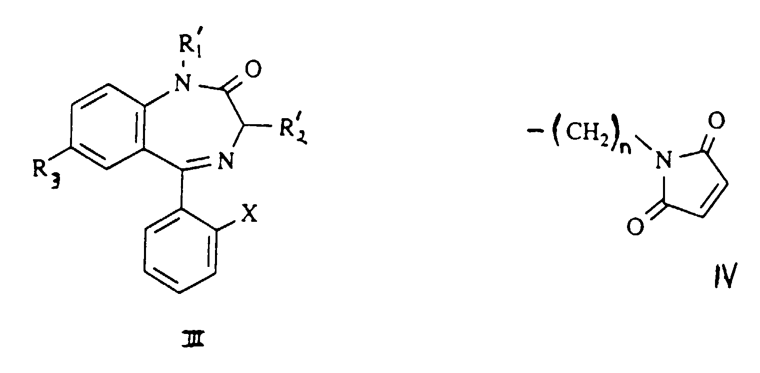 Die Benzodiazepin-Linkerverbindungen der Formel III sind neu. Ein weiterer Gegenstand der Erfindung sind daher diese Verbindungen und deren Verwendung zur Herstellung der erfindungsgemäßen Benzodiazepinproteinkonjugate.

Ein weiterer Gegenstand der Erfindung ist ein Verfahren zur Herstellung der Benzodiazepin-Linkerverbindungen der Formel III.

Ist in der Formel III R2' eine OR'-Gruppe und R1' eine Wasserstoff- oder CH3-Gruppe, so werden die Verbindungen folgendermaßen hergestellt:

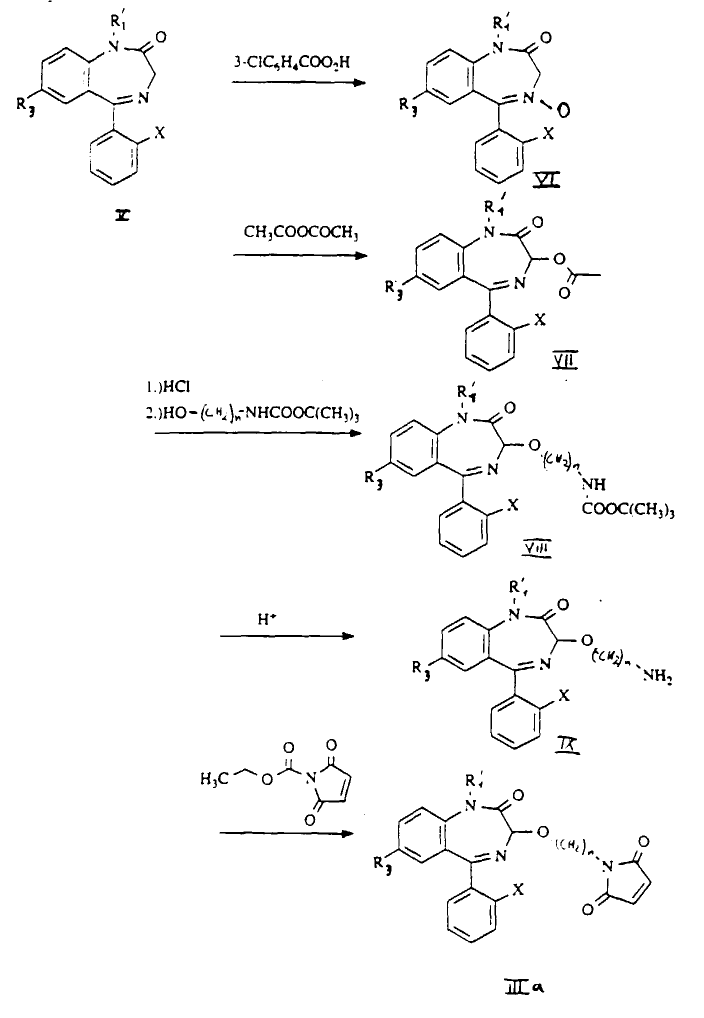

### (Schema 1)

Benzodiazepine der Formel V werden beispielsweise mit Metachlorperbenzoesäure zu Verbindungen der Formel VI oxidiert und mit Essigsäureanhydrid unter Umlagerung acyliert (Verbindungen der Formel VII). Solche Verbindungen werden in US-Patent 4,083,948 beschrieben. Anschließend wird unter Säurekatalyse mit N-geschütztem Ethanolamin oder Propanolamin umgesetzt (Verbindungen der Formel VIII und IX). Mit beispielsweise Ethoxycarbonylmaleimid wird anschließend zu den Benzodiazepin-3-Oxyethylmaleimiden bzw. Oxypropylmaleimiden der Formel III mit R2'=OR' umgesetzt.

Ist dagegen in Formel III R1' gleich R', so wird eine Benzodiazepinverbindung der Formel X mit N geschütztem Bromethylamin alkyliert und die Amidgruppe hydrolytisch zum Amin (Verbindung der Formel XI) gespalten. Die Bildung der 1-Ethylmaleimide der Formel III erfolgt beispielsweise durch Umsetzung mit Ethoxycarbonylmaleimid.

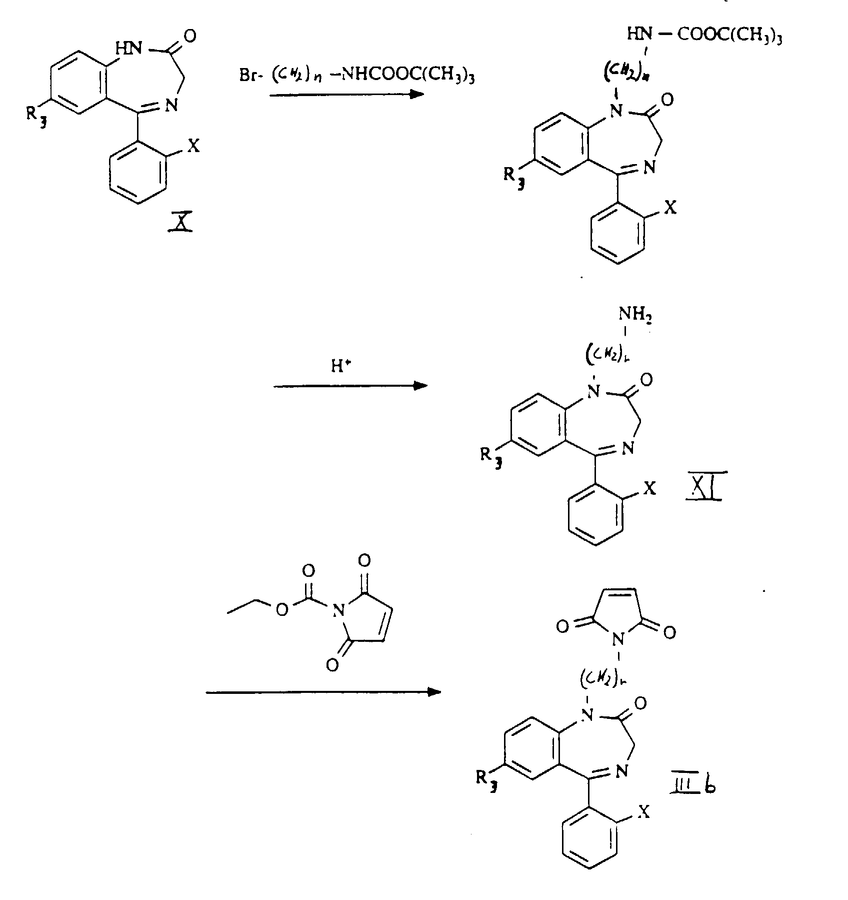

### Beispiel 1

### Immunisierung und Prüfung der Antiseren

5 Schafe werden mit Immunogenen, die durch Kopplung der Benzodiazepinverbindungen gemäß Beispiele 3 und 4 mit einer Trägersubstanz erhalten wurden, in Freund'schen Adjuvans immunisiert. Die Dosis beträgt 500 µg je Tier. Die Immunisierungen werden über 6 Monate oder länger jeweils in Abständen von 4 Wochen wiederholt.

Monatlich 1 x werden von allen Tieren Antiserum-Proben entnommen und auf Anwesenheit von Benzodiazepin-Antikörper untersucht. Die Durchführung dieser Messung ist in Beispiel 2 beschrieben. Für weitere Untersuchungen werden solche Antiseren ausgewählt, die in Verdünnung 1:10000 oder höher ausreichend hohes Meßsignal liefern (mindestens 100 mE nach 30 bis 60 Minuten Farbentwicklung).

Drei Monate nach Beginn der Immunisierung zeigten die Seren aller Tiere ausreichend hohes Signal.

### Beispiel 2

### Nachweis verschiedener Benzodiazepine

### Verwendete Lösungen

- Beschichtungspuffer:: 50 mM Natriumbicarbonat; 0,09% Natriumazid; pH 9,6
- Inkubationspuffer:: 10 mM Natriumphosphat; 0,1% Tween 20 (Fa. Brenntag, Best.Nr. 460761; 0,9 % NaCl; 1% Crotein C (Fa. CRODA GmbH, Best.Nr. 38241422); pH 7,4
- Waschlösung:: 0,9 % NaCl; 01,% Tween 20;
- Substratlösung:: Substratlösung Enzymun (Boehringer Mannheim GmbH, Best.Nr.85742), enthält 1,9 mM ABTS und 3,2 mM Natriumperborat in Phosphat-Citrat-Puffer pH 4,4) mit 2mg/ml Vanillin.

### Durchführung

### Beschichtung:

Mikrotiterplatten (Maxisorp F96, Fa. Nunc, Best. Nr. 4-42404) werden mit Streptavidin (Boehringer Mannheim GmbH, Best. Nr. 976 539) beschichtet, das in der Konzentration 5µg/ml Protein im Beschichtungspuffer gelöst wird. In jedem Napf der Mikrotiterplatten werden 100 µl dieser Lösung pipettiert. Nach einstündiger Inkubation bei Raumtemperatur unter Schütteln wird die Lösung ausgeschüttet und die Platte dreimal mit Waschlösung gewaschen.

### Synthese von Delorazepam-Biotinkonjugat (3)

92,4 mg (0,2 mmol) des Trifluoracetats von (2) werden in 10 ml THF gelöst und mit einer Lösung von 150.6 mg (0,24 mmol) Biotin-DDS (1) hergestellt (hergestellt nach WO-A 94/17072) in 5 ml DMF versetzt. Zum Reaktionsgemisch gibt man 50 µl Triethylamin und läßt 16 h bei 20°C rühren. Danach wird das Lösungsmittel am Rotationsverdampfer unter Ölpumpenvakuum entfernt und der Rückstand in 20 ml Chloroform aufgenommen. Man wäscht zweimal mit je 20 ml gesättigter NaHCO₃-Lösung und zieht anschließend das Chloroform am Rotationsverdampfer ab. Das feste Produkt 3 wird mit ca. 5 ml Essigester digeriert, abgesaugt und über Nacht im Exsikkator getrocknet.
- Ausbeute:: 21 mg (12% d. Th.) farbloses, feinkristallines Pulver
- DC:: Kieselgel, Essigester/Methanol 1/1 (v/v); R_{f}=0,48

Das Konjugat Delorazepam-1-DSS-Biotin (3) wird in Inkubationspuffer ad 10 ng / ml gelöst. Die Näpfe der Mikrotiterplatten werden mit je 100 µl dieser Lösung beschickt und ananschließend inkubiert und gewaschen wie oben beschrieben.

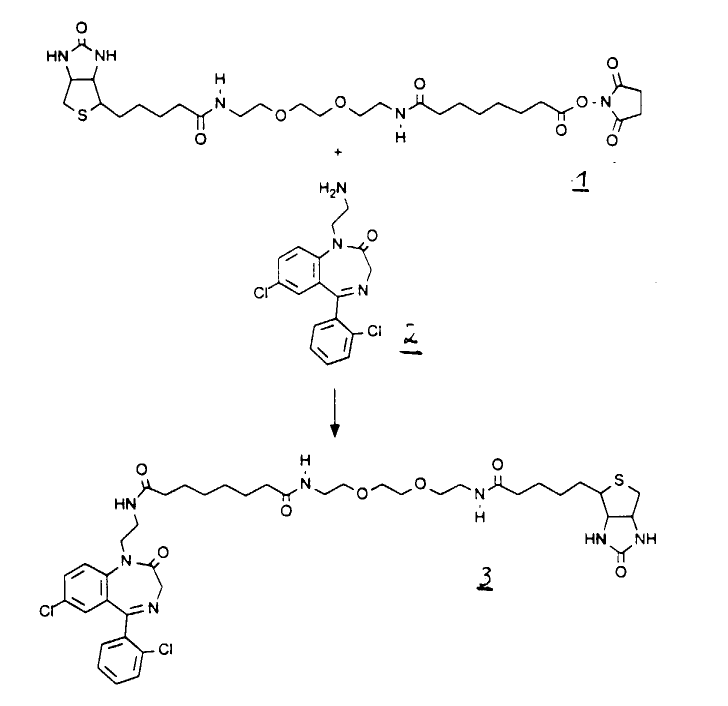

### Reaktion der Antikörper mit den Haptenen

Von jeder Benzodiazepin-Verbindung, die untersucht werden soll, wird eine Verdünnungsreihe in Inkubationspuffer hergestellt. Die Reihe enthält insgesamt 10 verschiedene Konzentrationen in Verdünnungsstufen 1:3, beginnend mit der Maximalkonzentration. Die Maximalkonzentrationen sind 1µg/ml für Temazepam, Oxazepam, Lorazepam, Bromazepam, Alprazolam, Hydroxy-Alprazolam, Hydroxy-Triazolam und Amino-Flunitrazepam. Die Maximalkonzentration beträgt 10 µg / ml für Temazepam-Glucuronid, 2-Amino-4-chlorbenzophenon, Oxazepam-Glucuronid, Lorazepam-Glucuronid und 2-Amino-2',4-dichlorbenzophenon.

Zum Vergleich wird Inkubationspuffer ohne Hapten eingesetzt. Je 50 µl dieser Lösungen werden in den Näpfen der Platte vorgelegt.

Die Antiseren werden mindestens 1 : 10000 mit Inkubationspuffer verdünnt. Je 50 µl des verdünnten Serums werden in die Näpfe mit den Hapten-Lösungen gegeben und gemischt. Die Endkonzentration der Haptene und der Antikörper im Test ist damit halb so hoch wie die der eingesetzten Lösungen.

Inkubation (60 Minuten) und waschen wie oben beschrieben.

### Reaktion mit dem Nachweis-Konjugat

Zum Nachweis der Antikörper, die über das Hapten-Biotin-Konjugat an die Festphase gebunden sind, dient ein Konjugat aus Meerrettich-Peroxydase und Kaninchen-Antikörpern gegen IgG aus Schafen. Das Nachweiskonjugat wird mit Inkubationspuffer auf 20 mU / ml Peroxidase-Aktivität verdünnt und diese Lösung auf die Näpfe verteilt (100 µl / Napf).

Inkubation (60 Minuten) und waschen wie oben beschrieben.

### Substratreaktion:

Alle Näpfe werden mit je 100 µl Substratlösung gefüllt und unter Schütteln inkubiert, bis die Farbentwicklung in den Proben ohne Hapten subjektiv ausreichend erscheint. Dann wird die Extinktion aller Näpfe als Differenzmessung bei denn Wellenlängen 405 / 492 Nanometer bestimmt.

### Auswertung

Als positives Ergebnis (eindeutiger Nachweis des eingesetzten Benzodiazepins) wird die Senkung des Meßsignals um mindestens 20% gegenüber dem Leerwert ohne Hapten betrachtet. Die Benzodiazepin-Konzentration, die genau diesem Signalwert entspricht (cut-off-Wert) wird durch lineare Interpolation zwischen benachbarten Standards der Hapten-Reihe für jede der untersuchten Verbindungen ermittelt.

### Ergebnisse:

Tabelle 1 zeigt die cut-off-Werte für eine Reihe von Benzodiazepin-Pharmaka und ihren Stoffwechsel-Derivaten, beispielhaft für die Antiseren von 3 oder 5 Schafen. Die Serumproben wurden 12 Monate nach Beginn der Immunisierung gewonnen.

Die Zahlen belegen, daß eine breite Palette von Benzodiazepinen völlig unterschiedlicher Struktur mit sehr guter bis ausreichender Sensitivität von allen drei Antiseren nachgewiesen werden kann.

**Tabelle 1**

| **Nachweis von Benzodiazepinen** | | | |
|---|---|---|---|
| Angegeben sind die Benzodiazepin-Konzentrationen (Nanogramm / ml), die im kompetitiven ELISA eine Signalerniedrigung um 20% gegenüber dem Leerwert ohne Hapten erzeugen. | | | |

| | **Tier-Nummer (eingesetzte Serumverdünnung)** | | |
|---|---|---|---|
| **Benzodiazepin-Verbindung** | **1** **(1:14000)** | **2** **(1:16500)** | **3** **(1:11500)** |
| Temazepam | 0,13 | 0,24 | 0,35 |
| 2-Amino-4-chlorbenzophenon | 64 | 68 | 69 |
| Temazepam-Glucuronid | 2,1 | n.b. | n.b. |
| Oxazepam | 0,29 | 0,26 | 0,41 |
| Oxazepam-Glucoronid | 7,5 | 8,7 | 11,0 |
| Lorazepam | 1,7 | 1,3 | 2,0 |
| Lorazepam-Glucuronid | 80 | 7,3 | 49 |
| 2-Amino-2',4-dichlorbenzophenon | 270 | 160 | 130 |
| Bromazepam | 5,5 | 150 | 25 |
| Alprazolam | 0,09 | 0,54 | 0,10 |
| Hydroxy-Alprazolam | 0,47 | 5,0 | 0,34 |
| Hydroxy-Triazolam | 3,0 | 15 | 9,1 |
| Amino-Flunitrazepam | 6,0 | 0,42 | 4,1 |

### Beispiel 3

### Synthese von 7-Chlor-3-[2-(N-maleinimido)ethyl]oxy-1-methyl-5-phenyl-1H-1,4-Benzodiazepin-2(3H)on (IIIa)

### 1. 7-Chlor-1-methyl-5-phenyl-1H-1,4-benzodiazepin-2(3H)on-4-oxid (VI)

5.70 g (20 mmol) 7-Chlor-1-methyl-5-phenyl-1H-1,4-benzodiazepin-2(3H)on (Fa.Sigma, Nr.T 8275) werden in 150 ml Dichlormethan gelöst und unter Rühren bei 20°C mit 13.8 g (40 mmol) 3-Chlor-peroxybenzoesäure (Fa.Aldrich, Nr.27,303-1) versetzt. Man läßt die Lösung 1.5 h bei 20°C weiterrühren. Danach wird die Reaktionsmischung mit 200 ml 5% NH3 und anschließend mit 200 ml 0.1 n NaOH gewaschen. Das Lösungsmittel wird am Rotationsverdampfer entfernt und der verbleibende ölige Rückstand unter Erwärmen auf 40-45°C in 25 ml Isopropanol gelöst. Danach läßt man das Produkt unter langsamen Abkühlen auf Raumtemperatur kristallisieren. 2h nach beginnender Kristallisation wird das feste Produkt 2 abgesaugt und mit je 50 ml Isopropanol und 50 ml Diisopropylether gewaschen. Man trocknet abschließend über Nacht im Exsikkator über Paraffin.
- Ausbeute:: 3.30 g (55% d.Th) farblose Kristalle.
- DC:: Kieselgel, Essigester/Petrolether 2/1 (v/v); Rf= 0.41

### 2. 3-Acetoxy-7-chlor-1-methyl-5-phenyl-1H-1,4-benzodiazepin-2(3H)on (VII)

3.0 g (10 mmol) der Verbindung VI werden 5 h in 30 ml THF + 60 ml Acetanhydrid unter Rückfluß erhitzt. Anschließend wird die Lösung am Rotationsverdampfer zur Trocknen eingedampft und der kristalline Rückstand mit 50 ml Isopropylether digeriert. Man saugt ab und trocknet das Produkt 3 2 h am Hochvakuum bei 50°C.
- Ausbeute:: 3.27 g (99 % d.Th.) farblose Kristalle.
- DC:: Kieselgel, Essigester/Petrolether 2/1 (v/v); Rf= 0.50

### 3. N-(tert.-Butoxycarbonyl)ethanolamin

30.5 g (0.5 mol) Ethanolamin (Fa.Fluka, Nr.02400) werden in 300 mlDioxan/Wasser 1/1 (v/v) gelöst und unter Eiskühlung tropfenweise mit einer Lösung von 109 g (0.5 mol) Di-tert.-butyldicarbonat (Fa.Fluka, Nr.34659) in 150 ml Dioxan versetzt. Man entfernt die Eiskühlung und läßt die Lösung unter Rühren auf Raumtemperatur kommen. Nach weiteren 18 h wird die Lösung am Rotationsverdampfer eingedampft, der ölige Rückstand in 250 ml Essigester gelöst und über ca.20 g Na2SO4 getrocknet. Nach Filtration wird abermals eingedampft und das ölige Produkt 3 h am Hochvakuum getrocknet.
- Ausbeute:: 80.2 g (99% d.Th.) farbloses Öl.
- DC:: Kieselgel, Essigester/Methanol 1/1 (v/v);
Detektion durch Besprühen mit Ninhydrin-Lösung; Rf= 0.74

### 4. 3-[2-(tert.-Butoxycarbonylamino)ethyl]oxy-7-chlor-1-methyl-5-phenyl-1H-1,4-benzodiazepin-2(3H)on (VIII)

1.5 g (5 mmol) der Verbindung VII werden 50 ml Chloroform (über Aluminiumoxid getrocknet) gelöst. Unter kräftigem Rühren bei 20°C leitet man 10 min über eine Kapillare in stetigem Strom (ca. 40-50 cm3/min) HCI-Gas durch die Lösung. Anschließend läßt man 18 h bei 20°C rühren. Dann wird die Reaktionslösung am Rotationsverdampfer zur Trocknung eingedampft. Der feste Rückstand wird in 20 ml Chloroform (über Aluminiumoxid getrocknet) gelöst und mit 960 mg (6 mmol) N-(tert.-Butoxycarbonyl)ethanolamin in 10 ml des gleichen Lösungsmittels versetzt. Man gibt 500 mg Nafion-NR 50 (Fa.Aldrich, Nr.30,938-9) zu und läßt 1 h bei 20°C rühren. Danach wird die Lösung filtriert, zweimal mit je 50 ml Wasser gewaschen und über ca. 5 g Na2SO4, wasserfrei, getrocknet. Das Lösungsmittel wird am Rotationsverdampfer entfernt. Dann wird das Rohprodukt in einem möglichst geringen Volumen Essigester/Petrolether 2/1 (v/v) unter leichtem Erwärmen gelöst und durch Säulenchromatographie an Kieselgel (Säule: 4 x 68 cm, Eluent: Essigester/Petrolether 2/1 (v/v)) gereinigt. Die entsprechenden Fraktionen werden gesammelt, das Lösungsmittel wird am Rotationsverdampfer entfernt und das kristalline Produkt 2 h im Hochvakuum bei 50°C getrocknet.
- Ausbeute:: 1.22 g (55 % d.Th.) farblose Kristalle.
- DC:: Kieselgel, Essigester/Petrolether 2/1 (v/v); Rf= 0.44

### 5. 3-(2-Aminoethyl)oxy-7-chlor-1-methyl-5-phenyl-1H-1,4-benzodiazepin-2(3H)on Hydrochlorid (IX)

1.11 g (2.5 mmol) der Verbindung VIII werden in 10 ml 2 molarer HCI in Dioxan gelöst, wobei sich innerhalb weniger Minuten ein öliger Niederschlag bildet. Man läßt die Suspension 30 min bei 20°C stehen, dekantiert das Lösungsmittel und digeriert den Rückstand mit 20 ml Essigester, wobei sich das Produkt verfestigt. Man rührt die Suspension 1 h bei 20°C, saugt ab und trocknet das Hydrochlorid 4 h bei 40°C am Hochvakuum.
- Ausbeute:: 910 mg (96% d.Th.) farbloses, feinkristallines Pulver.
- DC:: Kieselgel, n-Butanol/Eisessig/Wasser 50/15/25 (v/v/v); Rf= 0.64

### 6. 7-Chlor-3-[2-(N-maleinimido)ethyl]oxy-1-methyl-5-phenyl-1H-1,4-benzodiazepin-2(3H)on (IIIa)

760 mg (2 mmol) der Verbindung IX werden bei 20°C in 10 ml gesättigter Natriumbicarbonat-Lösung suspendiert und unter Rühren mit 340 mg (2 mmol) N-Methoxycarbonylmaleinimid (Fa.Fluka, Nr.64940) versetzt. Nach ca 2-3 min bildet sich ein öliger Niederschlag. Nach weiteren 10 min gibt man 15 ml Tetrahydrofuran zu und läßt weitere 45 min bei 20°C rühren. Danach stellt man die Reaktionsmischung mit konz. HCl auf pH 6.0, extrahiert mit 50 ml Essigester und wäscht den Extrakt mit 2 x 50 ml Wasser. Die organische Lösung wird mit ca 5 g Na2SO4 getrocknet, am Rotationsverdampfer eingedampft und das verbleibende Rohprodukt durch Säulenchromatographie an Kieselgel (Säule: 2.5 x 50 cm, Eluent: Essigester) gereinigt. Die entsprechenden Fraktionen werden gesammelt, das Lösungsmittel am Rotationsverdampfer entfernt und das verbleibende kristalline Maleinimid IIIa 3 h am Hochvakuum bei 40°C getrocknet.
- Ausbeute:: 315 mg (37% d.Th.) farbloser, feinkristalliner Feststoff.
- DC:: Kieselgel, Essigester: Rf= 0.49
1H-NMR(CDCl3): d(ppm) = 3.37(s,3H;-CH3), 3.91/4.14(m;4H;-CH2-CH2-), 4.87(s,1H;-C3H-), 6.69(s,2H;-CH=CH-), 7.48(m,8H; Aromaten-H).

### Beispiel 4

### Synthese von 7-Chlor-5-(2-Chlorphenyl)-1-[2-(N-maleinimido)ethyl]-oxy-1H-1,4-benzodiazepin-2(3H)on (IIIb)

### 1. N-(tert.-Butoxycarbonyl)-2-bromethylamin

10.2 g (50 mmol) 2-Bromethylamin Hydrobromid (Fa.Aldrich, Nr.B6,570-5) werden in 100 ml Dioxan/Wasser 1/1 (v/v) gelöst und mit 15 g (20.6 ml) Triethylamin versetzt. Man kühlt auf O° C und gibt unter Rühren tropfenweise eine Lösung von 10.9 g (50 mmol) Di-tert.-butyldicarbonat in 15 ml Dioxan zu. Die Reaktionsmischung läßt man langsam auf Raumtemperatur kommen und 18 h weiterrühren. Das Lösungsmittel wird am Rotationsverdampfer entfernt und der Rückstand in 200 ml Essigester aufgenommen. Man wäscht dreimal mit je 100 ml gesättigter Natriumbicarbonat-Lösung und abschließend zweimal mit je 100 ml Wasser. Die organische Lösung wird mit ca. 20 g Na2SO4 getrocknet, das Lösungsmittel am Rotationsverdampfer entfernt und das ölige Produkt 3 h bei 40°C am Hochvakuum getrocknet.
- Ausbeute:: 12.0 g (98 % d.Th.) leicht bräunliches, zähes Öl.
- DC:: Kieselgel, Essigester; Rf= 0.90

### 2. 7-Chlor-1-[2-(tert.-Butoxycarbonylamino)ethyl]-5-(2-chlorphenyl)-1H-1,4-benzodiazepin-2(3H)on

3.05 g (10 mmol) 7-Chlor-5-(2-chlorphenyl)-1H-1,4-benzodiazepin-2(3H)on (Fa.Sigma, Nr.D 7662) werden in 20 ml Dimethylformamid gelöst und unter Luftausschluß (N2-Atmosphäre) auf 0°C gekühlt. Die leicht gelbe Lösung wird mit 480 mg (20 mmol) Natriumhydrid (Suspension in Paraffinöl; Fa.Merck, Nr.818023) versetzt und 10 min bei 0°C gerührt. Dabei verfärbt sich die Reaktionsmischung nach Orange-Braun. Anschließend gibt man 2.69 (12 mmol) N-(tert.-Butoxycarbonyl)-2-bromethylamin zur Lösung und läßt allmählich auf Raumtemperatur kommen. Die Lösung wird 18 h bei 20°C gerührt, dann wird der Ansatz auf Eis gegossen. Der sich bildende Niederschlag wird abgesaugt und das Produkt 8 durch präparative Säulenchromatographie an Kieselgel (Säule: 4 x 68 cm, Eluent: Essigester) gereinigt. Die entsprechenden Fraktionen werden gesammelt, das Lösungsmittel am Rotationsverdampfer entfernt und der verbleibende Feststoff 1 h am Hochvakuum bei 40°C getrocknet.
- Ausbeute:: 2.66 g (59% d.Th.) farbloses, feinkristallines Pulver.
- DC:: Kieselgel, Essigester; Rf= 0.67

### 3. 1-[2-Aminoethyl]-7-chlor-5-(2-chlorphenyl)-1H-1,4-benzodiazepin-2(3H)on Trifluoracetat (XI)

2.24 g (5 mmol) der Verbindung aus 2. werden in 50 ml Essigester gelöst und auf 0°C gekühlt. Unter Rühren gibt man tropfenweise 10 ml Trifluoressigsäure zu und läßt ca. 30 min nachrühren. Danach werden Säure und Lösungsmittel am Rotationsverdampfer entfernt und das Produkt XI 5 h am Hochvakuum bei 40°C getrocknet.
- Ausbeute:: 2.32 g (100% d.Th.) leicht gelbliches, feinkristallines Pulver.
- DC:: Kieselgel, n-Butanol/Eisessig/Wasser 50/15/25 (v/v/v); Rf= 0.74 .

### 4. 7-Chlor-5-(2-chlorphenyl)-1-[2-(N-maleinimidoethyl]-1H-1,4-benzodiazepin-2(3H)on (IIIb)

1.16 g (2.5 mmol) des Trifluoracetats XI werden bei 20°C in 15 ml gesättigter Natriumbicarbonat-Lösung suspendiert und unter Rühren mit 425 mg (2.5 mmol) N-Methoxycarbonylmaleinimid in 20 ml Tetrahydrofuran versetzt. Man läßt 1 h bei 20°C rühren. Danach stellt man die Reaktionsmischung mit konz. HCI aufpH 6.0, extrahiert mit 50 ml Essigester und wäscht den Extrakt mit 2 x 50 ml Wasser. Die organische Lösung wird mit ca. 5 g Na2SO4 getrocknet, am Rotationsverdampfer eingedampft und das verbleibende Rohprodukt durch Säulenchromatographie an Kieselgel (Säule: 2.5 x 50 cm, Eluent: Essigester) gereinigt. Die entsprechenden Fraktionen werden gesammelt, das Lösungsmittel am Rotationsverdampfer entfernt und das verbleibende kristalline Maleinimid IIb 3 h am Hochvakuum bei 40°C getrocknet.
- Ausbeute:: 330 mg (31% d.Th.) farbloser, feinkristalliner Feststoff.
- DC:: Kieselgel, Essigester; Rf= 0.60
1H-NMR(CDCl3): d(ppm) = 3.86/4.10(m,4H;-CH2-CH2-), 3.80/4.86 (AB-Signal,J= 12Hz,2H;-C3H2-), 6.70(s,2H; -CH=CH-), 7.32(m,7H; Aromaten-H).

## Patentansprüche

1. Benzodiazepinproteinkonjugate der Formel I 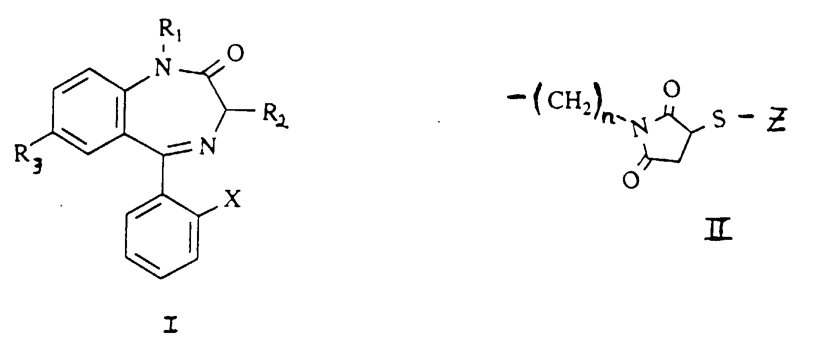 in der
R1 Wasserstoff eine Methylgruppe oder R darstellt,
R2 Wasserstoff, Hydroxy oder eine OR-Gruppe darstellt, wobei entweder R1 oder R2 eine R-Gruppe enthält,
R3 Halogen, NO2 oder NH2 darstellt,
X Wasserstoff oder Halogen darstellt,
wobei R eine Gruppe der Formel II darstellt, in der Z eine makromolekulare immunogen wirksame Trägersubstanz bedeutet, und n 2 oder 3 ist.

2. Benzodiazepinproteinkonjugate gemäß Anspruch 1 mit der Formel Ia oder Ib

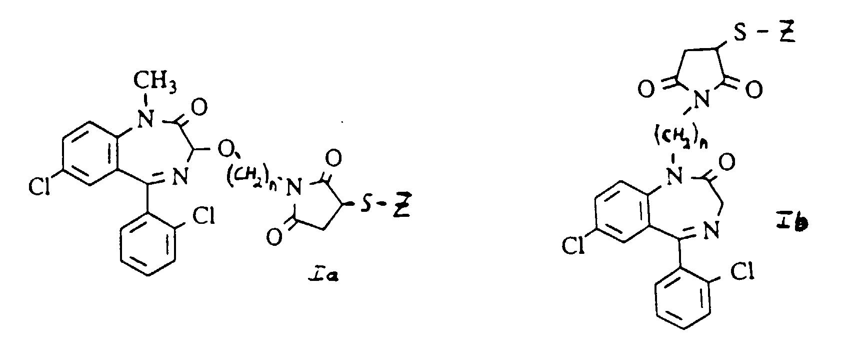

3. Verfahren zur Erzeugung von Antikörpern gegen Benzodiazepine durch Immunisierung eines Säugetieres und Gewinnung der erzeugten Antikörper nach bekannten Verfahren, dadurch gekennzeichnet, daß ein Konjugat gemäß Anspruch 1 oder 2 verwendet wird.

4. Antikörper, hergestellt nach einem Verfahren gemäß Anspruch 3.

5. Verfahren zur immunologischen Bestimmung der Anwesenheit von Benzodiazepinen oder Benzodiazepinmetaboliten in einer Probe durch Kontaktieren einer auf Benzodiazepine zu untersuchenden Probe mit einem Antikörper und Bestimmung des Antikörper-Benzodiazepin-Komplexes in einer geeigneten Weise, dadurch gekennzeichnet, daß als Antikörper ein Antikörper gemäß Anspruch 4 verwendet wird.

6. Verwendung der Konjugate gemäß Anspruch 1 oder 2 als Immunogene zur Herstellung von Antikörpern gegen Benzodiazepine.

7. Verwendung der Antikörper gemäß Anspruch 4 in Immunoassays.

8. Verfahren zur Herstellung der Benzodiazepinproteinkonjugate gemäß Anspruch 1 oder 2, dadurch gekennzeichnet, daß Benzodiazepinlinkerverbindungen der Formel III 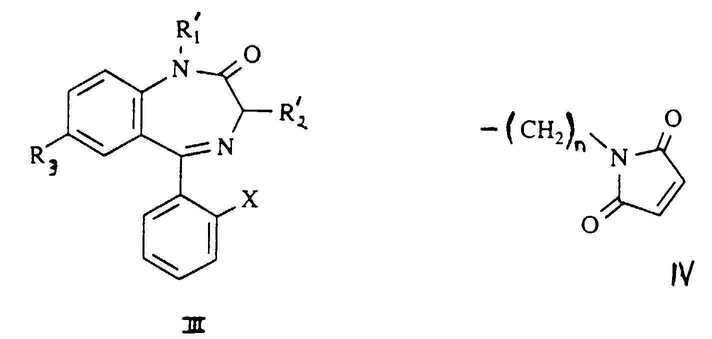 mit Polyaminoverbindungen, die mindestens eine Thiolgruppe enthalten, umgesetzt werden, wobei R1', Wasserstoff, Methyl oder R'darstellt, R'₂ ist Wasserstoff, OH oder OR' wobei entweder R1' oder R2' eine R' Gruppe enthält, R' eine Gruppe der Formel IV bedeutet, R³ Halogen, NO2 oder NH2 darstellt, X Wasserstoff oder Halogen darstellt.

9. Benzodiazepin-Linkerverbindungen der Formel III gemäß Anspruch 8.

10. Verfahren zur Herstellung der Benzodiazepinlinkerverbindungen der Formel III gemäß Anspruch 9, dadurch gekennzeichnet, daß
a) wenn R2' eine OR' Gruppe ist
Verbindungen der Formel V zu Verbindungen der Formel VI oxidiert, diese zu Verbindungen der Formel VII acyliert, mit N-geschütztem Alkylamin und Alkoxycarbonylmaleimid zu Verbindungen der Formel III umgesetzt wird 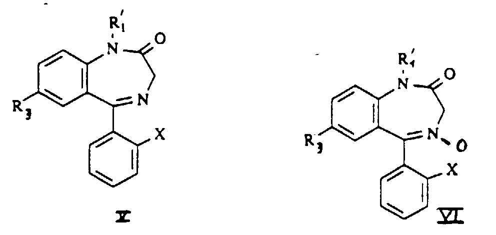 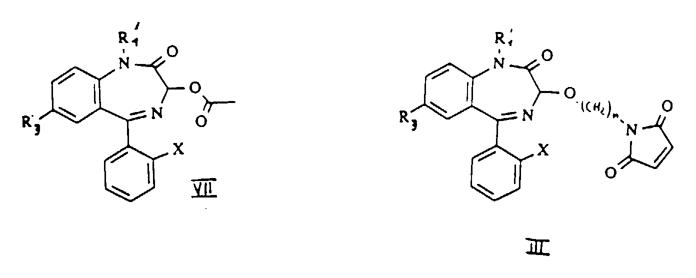 oder
b) wenn R1' eine R' Gruppe ist.
Benzodiazepine der Formel X werden mit N-geschütztem Halogenalkylamin alkyliert, die Schutzgruppe abgespalten und mit Alkoxycarbonylmaleimid zu Verbindungen der Formel III umgesetzt.

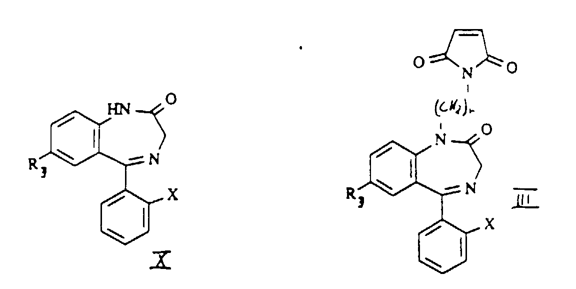

## Claims

1. Benzodiazepine-protein conjugates of the formula I 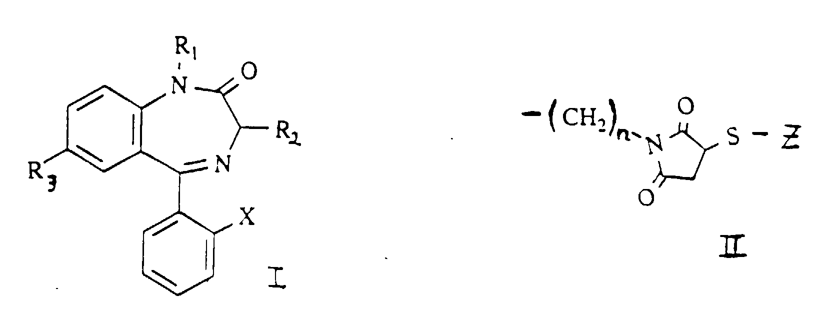 in which
R1 represents hydrogen, a methyl group or R,
R2 represents hydrogen, a hydroxy or an OR group, and either R1 or R2 contains an R group,
R3 represents halogen, NO2 or NH2,
X represents hydrogen or halogen,
wherein R represents a group of the formula II, in which Z is a macromolecular immunogenically active carrier substance, and n is 2 or 3.

2. Benzodiazepine protein conjugates as claimed in claim 1 having the formula 1a or 1b.

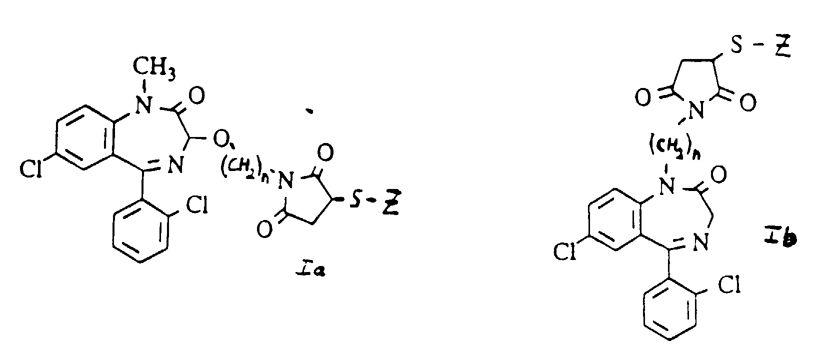

3. Method for producung antibodies to benzodiazepines by immunizing a mammal and isolating the produced antibodies by known methods, wherein a conjugate as claimed in claim 1 or 2 is used.

4. Antibody prepared according to a method of claim 3.

5. Method for the immunological determination of the presence of benzodiazepines or benzodiazepine metabolites in a sample by contacting a sample to be examined for benzodiazepines with an antibody and determining the antibody-benzodiazepine complex in a suitable manner, wherein an antibody as claimed in claim 4 is used as the antibody.

6. Use of the conjugates as claimed in claim 1 or 2 as immunogens for the preparation of antibodies to benzodiazepines.

7. Use of the antibodies as claimed in claim 4 in immunoassays.

8. Process for producing benzodiazepine-protein conjugates as claimed in claim 1 or 2, wherein benzodiazepine linker compounds of formula III 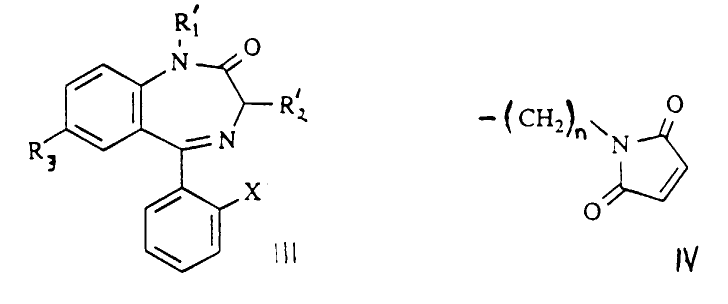 are reacted with polyamino compounds containing at least one thiol group, wherein R1' represents hydrogen, methyl or R', R'₂ is hydrogen, OH or OR' and either R1' or R2' contains an R' group, R' denotes a group of the formula IV, R3 represents halogen, NO2 or NH2, and X represents hydrogen or halogen.

9. Benzodiazepine linker compounds of the formula III as claimed in claim 8.

10. Process for producing benzodiazepine linker compounds of formula III as claimed in claim 9, wherein
a) if R2' is an OR' group, compounds of the formula V are oxidized to compounds of formula VI, these are acylated to form compounds of formula VII and reacted with N-protected alkylamine and alkoxycarbonyl-maleimide to form compounds of the formula III 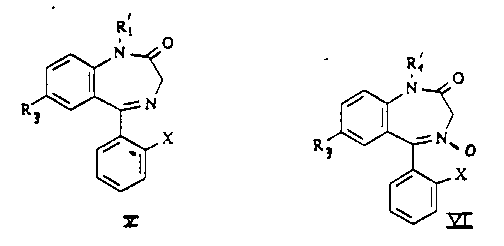 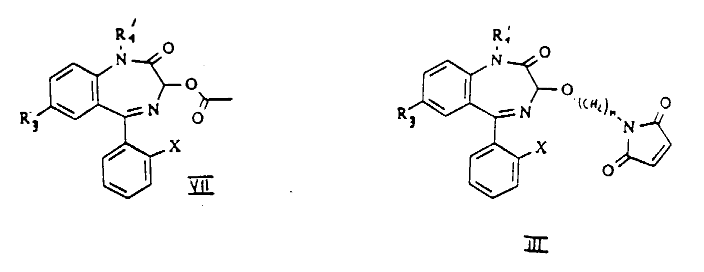 or
b) if R1' is an R' group,
benzodiazepines of formula X are alkylated with N-protected halogenalkylamine, the protective group is cleaved off and they are reacted with alkoxycarbonylmaleimide to form compounds of the formula III.

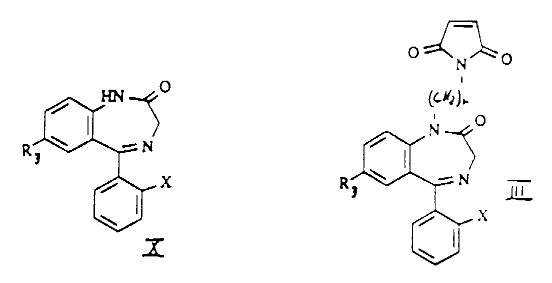

## Revendications

1. Produits de conjugaison de benzodiazépines-protéines, répondant à la formule I 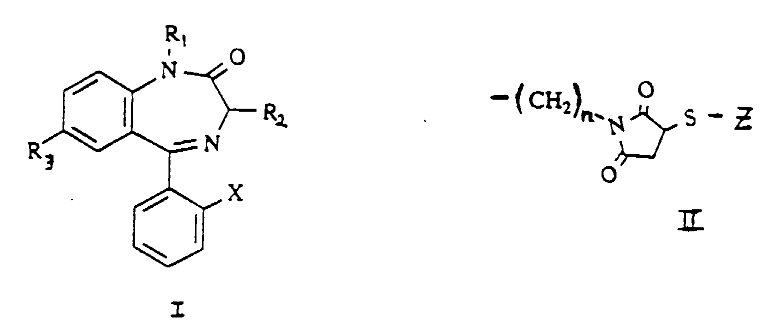 dans laquelle
R₁ représente un atome d'hydrogène, un groupe méthyle ou R,
R₂ représente un atome d'hydrogène, un groupe hydroxyle ou un groupe OR, soit R₁, soit R₂ contenant un groupe R,
R₃ représente un atome d'halogène, un groupe NO₂ ou un groupe NH₂,
X représente un atome d'hydrogène ou un atome d'halogène,
R représentant un groupe de formule II dans laquelle Z désigne une substance de support macromoléculaire à activité immunogène et n est égal à 2 ou 3.

2. Produits de conjugaison de benzodiazépines-protéines selon la revendication 1, répondant à la formule la ou à la formule Ib

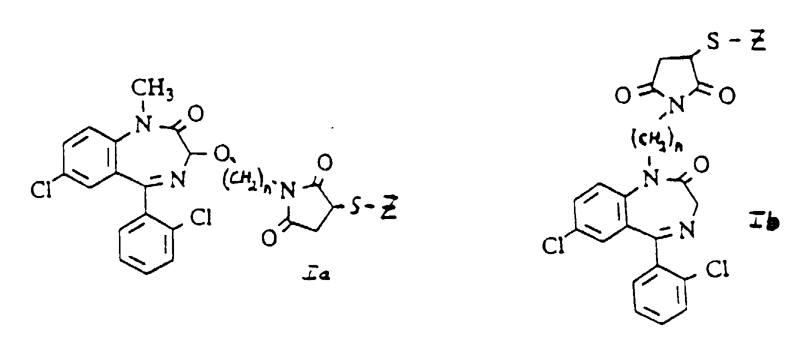

3. Procédé pour la formation d'anticorps contre des benzodiazépines par immunisation d'un mammifère et récupération des anticorps obtenus, conformément à des procédés connus, caractérisé en ce qu'on utilise un produit de conjugaison selon la revendication 1 ou 2.

4. Anticorps préparés conformément à un procédé selon la revendication 3.

5. Procédé pour la détermination immunologique de la présence de benzodiazépines ou de métabolites des benzodiazépines dans un échantillon par mise en contact d'une sonde destinée à détecter des benzodiazépines avec un anticorps et détermination du complexe anticorps-benzodiazépines d'une manière appropriée, caractérisé en ce qu'on utilise, à titre d'anticorps, un anticorps selon la revendication 4.

6. Utilisation des produits de conjugaison selon la revendication 1 ou 2, à titre d'immunogènes pour la préparation d'anticorps contre des benzodiazépines.

7. Utilisation des anticorps selon la revendication 4, dans des immunodosages.

8. Procédé pour la préparation des produits de conjugaison de benzodiazépines-protéines selon la revendication 1 ou 2, caractérisé en ce qu'on fait réagir des composés de liaison à des benzodiazépines répondant à la formule III 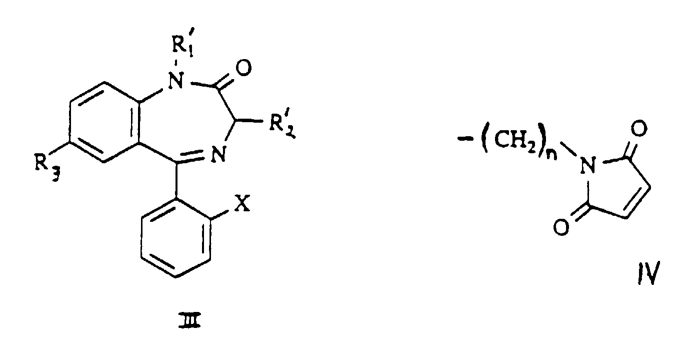 avec des composés polyamino qui contiennent au moins un groupe thiol, dans laquelle R'₁ représente un atome d'hydrogène, un groupe méthyle ou R', R'₂ représente un atome d'hydrogène, un groupe OH ou un groupe OR', soit R'₁, soit R'₂ contenant un groupe R', R' représentant un groupe de formule IV, R₃ représente un atome d'halogène, un groupe NO₂ ou un groupe NH₂, X représente un atome d'hydrogène ou un atome d'halogène.

9. Composés de liaison à des benzodiazépines répondant à la formule III selon la revendication 8.

10. Procédé pour la préparation des composés de liaison à des benzodiazépines répondant à la formule III selon la revendication 9, caractérisé en ce que
a) lorsque R'₂ représente un groupe OR',
on oxyde des composés de formule V pour obtenir des composés de formule VI, on soumet ces derniers à une acylation pour obtenir des composés de formule VII que l'on fait réagir avec des alkylamines et des alcoxycarbonylmaléimides protégés sur l'atome d'azote pour obtenir les composés de formule III 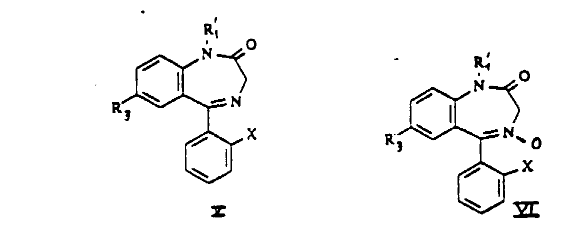 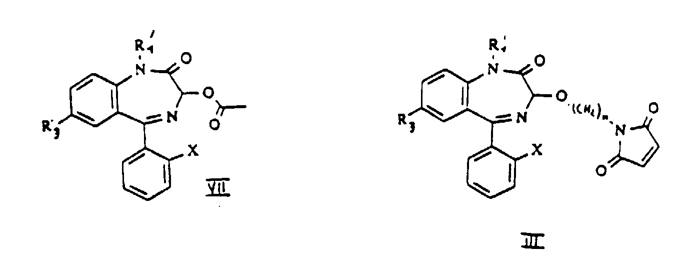 ou
b) lorsque R'₁ représente un groupe R',
on soumet des benzodiazépines de formule X à une alkylation avec des halogénoalkylamines protégées sur l'atome d'azote, on élimine le groupe de protection et on fait réagir avec des alcoxycarbonylmaléimides pour obtenir des composés de formule III.
